Europäisches Patentamt

European Patent Office

Office européen des brevets

(11) Veröffentlichungsnummer: **0 234 492**
**A2**

(12) # EUROPÄISCHE PATENTANMELDUNG

(21) Anmeldenummer: **87102293.5**

(22) Anmeldetag: **18.02.87**

(51) Int. Cl.⁴: **G01N 21/55** , G01N 21/17 , G01N 21/47

(30) Priorität: **22.02.86 EP 86102332**

(43) Veröffentlichungstag der Anmeldung:
**02.09.87 Patentblatt 87/36**

(84) Benannte Vertragsstaaten:
**AT BE CH DE ES FR GB IT LI NL SE**

(71) Anmelder: **Helmut K. Pinsch GmbH & Co.**
**Hansastrasse 13**
**D-2000 Hamburg 13(DE)**

(72) Erfinder: **Hatje, Günter H.**
**Wellingsbüttler Weg 182**
**D-2000 Hamburg 65(DE)**

(74) · Vertreter: **Patentanwälte Dipl.-Ing. J. Richter**
**Dipl.-Ing. F. Werdermann**
**Neuer Wall 10**
**D-2000 Hamburg 36(DE)**

(54) **Prüfvorrichtung und Verfahren für die Erfassung unterschiedlich ausgetalteter Oberflächen von Gegenständen.**

(57) Bei einer Prüfvorrichtung für die Erfassung von Gegenständen, die unterschiedlich ausgestaltete Oberflächen aufweisen können und die relativ zu der Prüfvorrichtung bewegt werden, ist es vorgesehen, daß der zu prüfende Gegenstand (12) in einer Ebene quer zur Hauptbewegungsrichtung (Pfeil 14) mit im wesentlichen gleicher Intensität über einen schmalen und sich über die Breite des Gegenstandes erstrekkenden Bereich beleuchtet wird. Eine die reflektierten Helligkeits-und/oder Farbwerte des Gegenstandes erfassende Erfassungsvorrichtung gibt ansprechend auf Änderung der erfaßten Werte in der beleuchteten Ebene ein Informationssignal ab, das von einem Rechner interpretiert wird.

FIG 1

EP 0 234 492 A2

## Prüfvorrichtung und Verfahren für die Erfassung unterschiedlich ausgestalteter Oberflächen von Gegenständen.

Die Erfindung betrifft eine Prüfvorrichtung und ein Verfahren für die Erfassung unterschiedlich ausgestalteter Oberflächen von Gegenständen, wie keramische Erzeugnisse, Gegenstände aus Glas, Holz, Kunststoffen, in Klarsichtfolie verpackte Gegenstände u.dgl.

Unter derartigen Prüfvorrichtungen sind beispielsweise solche für Holz, insbesondere Schnittholz, bekannt geworden. Bei bekannten Schnittholz-Prüfvorrichtungen wird einseitig ein mechanischer Impuls an das zu prüfende Schnittholz-Brett abgegeben. Mittels mehrerer Sensoren, die in vorbestimmten Abständen an dem Brett angebracht sind, wird der Amplitudenverlauf der dadurch erzeugten Stoßwelle gemessen. Die von den Sensoren abgegebenen Daten werden in einen Prozeßrechner eingegeben, der aus den gemessenen Durchlaufzeiten den E-Modul zwischen den Sensoren ermittelt. Aus Abweichungen von den Standardwerten kann auf das Vorliegen eines Holzfehlers in dem Brett geschlossen werden.

Dieses Verfahren läßt sich jedoch nur bei Brettern anwenden, die die gleiche Länge haben. Soll die Produktion für die nächste Charge auf andere Längen umgestellt werden, ist jeweils eine relativ aufwendige Anpassung erforderlich.

Zudem können mit diesem bekannten Verfahren nicht alle auftretenden Holzfehler in gleicher Weise zuverlässig erfaßt werden. Hierzu gehören Farbfehler, die nicht erfaßt werden können. Ein weiterer Nachteil besteht darin, daß die Sensoren an jedem zu messenden Brett erneut angebracht werden müssen. Ein kontinuierlicher Betrieb ist unmöglich.

Ferner ist es bekannt, mittels eines optischen Erfassungssystem, das auf einer handelsüblichen Fernsehkamera beruht, ein Bild zeilenweise zu erfassen und abzuspeichern. Die abgespeicherten Bilddaten werden dann einem Rechner zugeleitet, der sie einer besonderen ikonischen und/oder symbolorientierten Verarbeitung unterwirft.

Bei einem derartigen Verfahren ist nachteilig, daß für eine hohe Auflösung auch eine entsprechend hohe Speicherkapazität benötigt wird. Dieser Nachteil ist besonders gravierend, wenn unterschiedliche Arten von Bildern abgespeichert werden müssen, da dann für jedes Bild ein separater Speicherbereich reserviert werden muß.

Der Erfindung liegt die Aufgabe zugrunde, eine Prüfvorrichtung und ein Verfahren zum Erfassen unterschiedlich ausgestalteter Oberflächen von Gegenständen zu schaffen, mit denen auftretende Besonderheiten der zu erfassenden Gegenstände - schnell und zuverlässig erfaßt und ermittelt werden können, ohne daß aufwendige Vorkehrungen zur Abspeicherung erforderlich werden.

Zur Lösung dieser Aufgabe wird eine Prüfvorrichtung zum Erfassen unterschiedlich ausgestalteter Oberflächen von Gegenständen gemäß der eingangs beschriebenen Art vorgeschlagen, die erfindungsgemäß in der Weise ausgebildet ist, daß der zu prüfende Gegenstand relativ zu der Prüfvorrichtung in einer Hauptbewegungsrichtung bewegbar ist, daß der Gegenstand in einer Ebene quer zur Hauptbewegungsrichtung mit im wesentlichen gleicher Intensität beleuchtbar ist und daß eine in dieser Ebene angeordnete, die reflektierten Helligskeits-und/oder Farbwerte des Gegenstandes in Zeilenform erfassende optische Erfassungsvorrichtung, ansprechend auf Helligkeits-und/oder Farbänderungen in der von der Erfassungsvorrichtung erfaßten beleuchteten Ebene des Gegenstandes, während der Relativbewegung von Gegenstand und optischer Erfassungsvorrichtung ein Informationssignal abgibt.

Des weiteren wird diese Aufgabe durch ein Erfassungsverfahren gelöst, nach dem erfindungsgemäß die Gegenstände kontinuierlich in einer Hauptbewegungsrichtung relativ zu einer optischen Erfassungsvorrichtung durch Absolutbewegung der optischen Erfassungsvorrichtung oder der Gegenstände bewegt werden, wobei ein schmaler und sich über die Breite des Gegenstandes erstreckender Bereich in einer sich quer zur Hauptbewegungsrichtung erstreckenden Ebene beleuchtet wird und wobei die optische Erfassungsvorrichtung in der Ebene quer zur Bewegungsrichtung des Gegenstandes arbeitet und ansprechend auf Helligkeits-und/oder Farbänderungen des Gegenstandes während der Relativbewegung von Gegenstand und optischer Erfassungsvorrichtung ein Informationssignal abgibt.

Besonders vorteilhaft ist es, daß durch Echtzeiterfassung ein kontinuierlicher Betrieb der Prüfvorrichtung und damit ein Online-Einsatz möglich ist. Bei Verwendung z.b. von Schnittholz als zu prüfendem Gegenstand können auftretende Holzfehler, z.B. Astknorren oder -wurzeln, Rotfäule od.dgl. schnell und zuverlässig erfaßt werden. Darüber hinaus können Fehlstellen, stück-und lagebezogen, erfaßt werden, so daß geeignete Abschnitte mit bestimmter und gewünschter fehlerfreier Länge ermittelt werden können, um Schnittholz geringerer Güteklasse für höherwertige Zwecke verwenden zu können.

Die optische Erfassungsvorrichtung ist mit einer Rechenvorrichtung verbunden. Letztere weist eine Speichervorrichtung auf, in der die auftretenden Informationssignale abspeicherbar sind.

Dadurch, daß ein optisches Verfahren verwendet wird, kann zunächst der Vorteil erreicht werden, daß auch Farbfehler registriert werden können. Es besteht nämlich die Möglichkeit, ansprechend auf Helligkeitsunterschiede oder auf Farbunterschiede oder auf eine Kombination von Helligkeits-und Farbunterschieden ein Informationssignal abzugeben, das die Lage der jeweiligen Besonderheit kennzeichnen kann. Als Besonderheit kommt z.B. eine Fehlstelle in einem Stück Schnittholz, ein Sprung in einer Fliese oder Kachel, ein Farbfehler bei dieser, eine Verfärbung eines in Klarsichtfolie verpackten Produktes od.dgl. infrage. Da keine Sensoren an jedem Gegenstand angelegt werden müssen, ist die Realisierung eines kontinuierlichen Betriebes möglich. Beispielsweise kann die Prüfvorrichtung feststehend ausgebildet sein und die Gegenstände an der Prüfvorrichtung vorbeibewegt werden. Holz z.B. kann so mit einer Geschwindigkeit von 3 m/s geprüft werden.

Ein besonderer Vorteil besteht darin, daß mit der Vorrichtung trotz kontinuierlichen Betriebes die Erfassung von Fehlstellen stückbezogen erfolgen kann. Es ist sogar möglich, zu jedem gelagerten Gegenstand, insbesondere zu jedem Stück Holz, neben der absoluten Länge die fehlerfreie Länge, also die Länge zwischen etwaigen Fehlstellen, abzuspeichern. Wenn nun Schnittholz mit einer bestimmten fehlerfreien Länge verlangt wird, so kann das hierfür geeigneteste Stück Schnittholz schnell ermittelt werden. Das gleiche trifft auch auf die Überprüfung von Holzfurnieren zu.

Praktisch wird es so möglich, auch Schnittholz aus einer nach DIN 68256 vorgegebenen geringeren Güteklasse für höherwertige Zwecke zu verwenden.

Erfindungsgemäß kann auch die bislang durchgeführte Sichtkontrolle der zu prüfenden Gegenstände, z.B. des Holzes, die das Auge des Prüfenden stark belastete, völlig entfallen.

Vorteilhafte Weiterbildungen ergeben sich aus den Unteransprüchen.

Durch Verbindung der optischen Erfassungsvorrichtung und der Rechenvorrichtung ist das abgegebene Informationssignal interpretierbar. Die Rechenvorrichtung wird so ausgewählt, daß sie eine ausreichende Geschwindigkeit aufweist, um die eingehenden Informationssignale in Echtzeit zu verarbeiten. Zweckmäßigerweise, jedoch nicht notwendigerweise, werden die auftretenden Informationssignale sequentiell in einer Speichervorrichtung abgespeichert.

Des weiteren betrifft die Erfindung eine Ausgestaltung, bei der die Rechenvorrichtung eine Zeit- oder Längenmeßeinrichtung aufweist, die die Zeitdauer oder die Länge vom Erfassen der Vorderkante eines Gegenstandes, gesehen in der Hauptbewegungsrichtung, bis zum Auftreten des Informationssignales mißt, wobei die Zeit-oder Längenmeßeinrichtung mit der Speichervorrichtung verbunden ist.

Gemäß einer weiteren Ausgestaltung der Erfindung ist eine Anpaßschaltung (Interface) vorgesehen, mit der eine Erkennungsschwelle für das Informationssignal einstellbar ist. Die Anpaßschaltung kann außerdem, insbesondere elektronische, Steuerelemente für die Anpassung der optischen Erfassungsvorrichtung oder der Lichtquelle an unterschiedlichen Grundhelligkeiten der Oberfläche der geprüften Gegenstände oder der Lichtquelle unter Ausnutzung der maximalen Dynamik der optischen Erfassungsvorrichtung aufweisen.

Die optische Erfassungsvorrichtung weist ferner zweckmäßigerweise einen Filter für die Erhöhung der Empfindlichkeit auf bestimmte Besonderheiten der zu prüfenden Gegen stände, die erfaßt werden sollen, auf.

Die optische Erfassungsvorrichtung mit der Lichtquelle ist an einem Tragrahmen angeordnet, der z.B. ringförmig die zu erfassenden Gegenstände umgibt, wobei je nach der Erfassungsfläche an dem Tragrahmen oben und/oder unten und/oder an den Seiten optische Erfassungsvorrichtungen mit Lichtquellen angeordnet sein können. Es besteht auch die Möglichkeit, nur eine einzige Lichtquelle zu verwenden, wobei dann bei dieser Ausführungsform an dem Tragrahmen eine entsprechende Anzahl von Umlenkspiegeln derart angeordnet sind, daß nur bestimmte Flächen des zu erfassenden Gegenstandes oder alle Flächen des Gegenstandes beleuchtet werden. Dies gilt auch für die optische Erfassungsvorrichtung, die über Umlenkspiegel auf die zu erfassende Fläche oder die zu erfassenden Flächen gerichtet ist, so daß neben einer der Anzahl der zu erfassenden Flächen entsprechenden Anzahl von optischen Erfassungsvorrichtungen auch nur eine einzige optische Erfassungsvorrichtung zum Einsatz gelangen kann. Dann können die unterschiedlichen Lichtintensitäten der beleuchteten Flächen als Unterscheidungsmerkmal dafür herangezogen werden, welche der Flächen während des Augenblicks des Erfassens gerade erfaßt wird, um das erforderliche Informationssignal zu erhalten. Ferner besteht auch die Möglichkeit, die verschiedenen Flächen des Gegenstandes nacheinander in einem vorgegebenen Zeitraster zu erfassen und die erhaltenen Werte abzuspeichern.

Besonders vorteilhaft ist es, daß die Abtastung in Zeilen erfolgt. Die Zeile erstreckt sich quer zur Hauptbewegungsrichtung des Gegenstandes. Wenn innerhalb einer Zeile eine Fehlstelle erfaßt wird, führt dies zur Abgabe eines Informationssignales. Zusätzlich wird die Position der Fehlstelle, z.B. bezogen auf die Längsrichtung des Holzes, abgespeichert. Auf einfache Weise kann aus der zwischen zwei erfaßten Fehlstellen zurückgelegten Wegstrecke die fehlerfreie Länge ermittelt werden.

Besonders vorteilhaft ist es ferner, wenn für die optische Abtastung eine CCD-Meßzeile verwendet wird. Die Ausgangssignale dieser Meßzeile können leicht in einem Rechner aufbereitet und abgespeichert werden. Hierbei kann ein handelsübliches Interface verwendet werden. Ferner ist auch die Helligkeits-Grundeinstellung und die Autofokussierung relativ einfach zu bewerkstelligen. Besonders vorteilhaft ist es, wenn lediglich die Fehlerschwelle, d.h. der Helligkeitsunterschied, ab welchem eine Helligkeitsänderung als Fehler erfaßt werden soll, eingestellt werden muß.

Gemäß einer weiteren vorteilhaften Ausgestaltung ist es vorgesehen, eine handelsübliche Farb-Videokamera zu verwenden. Eine derartige Kamera gibt ein Abtastsignal in einer bereits in Zeilen aufbereiteten Form ab. Dieses kann der Recheneinrichtung zugeleitet werden und online verarbeitet werden. Auch Gegenstände mit glänzenden Oberflächen können hierbei geprüft werden.

Es ist beispielsweise möglich, Klarsichtpackungen auf Produkt-und Verpackungsfehler zu prüfen.

Die Einpegelung auf die Helligkeit von fehlerfreien Gegenständen kann beispielsweise dadurch erfolgen, daß zunächst in einer Einstell-Betriebsart des Rechners ein fehlerfreier Gegenstand durch die Prüfvorrichtung geschickt wird. Dabei wird dann der durchschnittliche Helligkeitswert erfaßt und als Referenz für die weiteren Messungen verwendet.

Gewünschtenfalls kann auch die Lage der Fehlstelle, bezogen auf die Achse der CCD-Zeile od.dgl., mit abgespeichert werden.

Ferner ist es möglich, die Gegenstände von vier Seiten zu begutachten, indem vier erfindungsgemäße Prüfvorrichtungen links, rechts, oberhalb und unterhalb des zu prüfenden Gegenstandes, wie z.B. Schnittholz, angeordnet werden. Dadurch, daß der Gegenstand an zwei der vier Seitenflächen anliegen kann, ist lediglich die Verwendung von zwei Autofokussiereinrichtungen an den beiden anderen Prüfeinrichtungen erforderlich.

Ein weiterer besonderer Vorteil der Erfindung besteht darin, daß Gegenstände wie Holz oder Walzstahl in nahezu beliebiger Länge und Stärke geprüft werden können, daß ferner keine Einschränkungen hinsichtlich der verwendeten Holzart bestehen und daß auch die bislang nur durch Inaugenscheinnahme erfaßbaren Farbfehler abgespeichert werden können.

Besonders vorteilhaft ist es ferner, daß die gemessenen Daten auch in einem integrierten System abgespeichert werden können, in welchem neben der Abspeicherung der Fehlstelle, der Qualität, der Abmessungen und des Lagerortes eines jeden Holzstückes auch die für die kaufmännische Abwicklung erforderlichen Maßnahmen durchgeführt werden können.

Die erfindungsgemäße Prüfvorrichtung läßt sich in vorteilhafter Weise an automatisierten Fertigungsanlagen einsetzen. Beispielsweise kann der Arbeitsraum von Robotern überwacht werden, um festzustellen, ob sich ein sich bewegendes Gut in dem Arbeitsraum befindet. Zugleich kann eine Sicherheitsabschaltung vorgenommen werden, falls es sich bei dem erfaßten Objekt um eine Person handelt.

In einer Produktionslinie hergestellte Gegenstände können auf genaue Position von Formmerkmalen, wie Bohrungen und Aussparungen, überwacht werden. Ferner ist es auch möglich, Gegenstände aus durchsichtigen Materialien, wie Fernsehröhren, Trinkgläser od.dgl. bei der Produktion zu überwachen. Auch können Gegenstände mit polierter und sogar glänzender Oberfläche, wie Hammerschlaglackierung, geprüft werden.

Besonders vorteilhaft kann die zu erfassende Oberfläche schräg mit Licht beaufschlagt werden. Damit ist es möglich, Oberflächenunebenheiten aufgrund der Schattenbildung zu erfassen.

Farbfehler von Kacheln od.dgl. können durch Farbwerterfassung über Videokameras im Vergleich mit einem vorgegebenen Ist-Farbbild durchgeführt werden, wobei es möglich ist, fehlerhafte Kacheln bereits unmittelbar nach dem Zeitpunkt der Erfassung auszusondern oder aber aufgrund der erfaßten Informationssignale in unterschiedliche Qualitätskategorien einzureihen.

Zweckmäßig ist hierbei eine gruppenbildende Erfassung der Informationssignale durch den Rechner, wobei eine Ungleichmäßigkeit der Struktur erkannt werden kann, ohne daß extrem schnelle Rechner erforderlich wären.

Durch die Echtzeit-Verarbeitungsmöglichkeit ist es wahlweise möglich, die erfaßten Signale in Echtzeit unmittelbar, d.h. ohne Zwischenspeicherung, zu verarbeiten, oder diese Signale zwischenzuspeichern und in einem nachfolgenden Schritt diese in vorzugsweise gleicher Geschwindigkeit auszulesen und in der erforderlichen Weise umzuformen. Dabei kann auch das an sich bekannte Pipelining angewendet werden, und es können jeweils dezidierte Rechner für die erforderlichen Zwecke eingesetzt werden.

Beispielsweise ist es möglich, innerhalb einer rechnergesteuerten Produktionsanlage die Erfassung durch FrontEnd-Prozessoren vornehmen zu lassen, hier zugleich eine Datenreduktion vorzunehmen und die erfaßten Informationssignale von mehreren Front-End-Prozessoren einem zentralen Rechner zuzuleiten, der in geeigneter Form die Produktionssteuerung vornimmt.

Weitere Einzelheiten, Merkmale und Vorteile werden anhand mehrerer Ausführungsbeispiele in der Zeichnung näher erläutert. Es zeigt

Fig. 1 eine perspektivische Ansicht einer ersten Ausführungsform einer Prüfvorrichtung, wobei beispielhaft eine solche für Schnittholz beschrieben wird,

Fig. 2 ein Blockschaltbild einer Ausführungsform einer Meßschaltung für eine Prüfvorrichtung gemäß Fig. 1,

Fig. 3 ein Blockschaltbild einer weiteren Ausführungsform der Meßschaltung,

Fig. 4 eine schematische Darstellung eines Teils der Prüfvorrichtung gemäß Fig. 1,

Fig. 5 eine Ansicht einer weiteren Ausführungsform dieser Prüfvorrichtung und

Fig. 6 Zeitdiagramme zur Darstellung von Meßsignalen, die in der Meßschaltung gemäß Fig 3 auftreten.

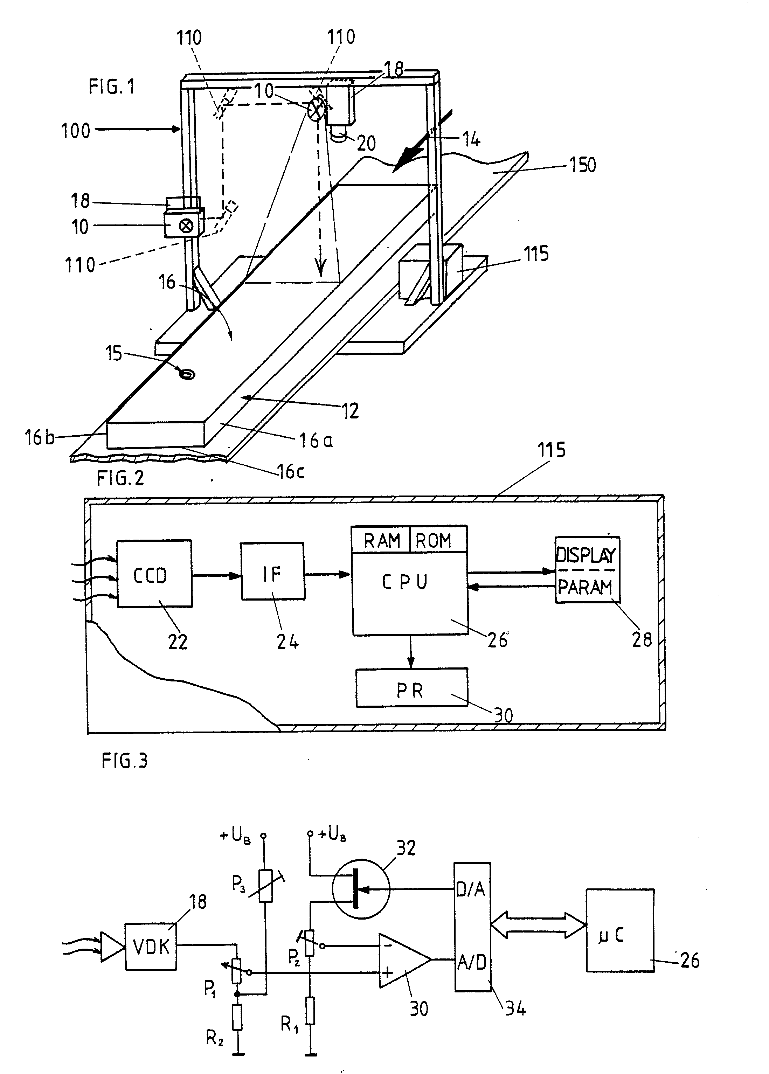

Bei der in Fig. 1 dargestellten Ausführungsform ist eine Lichtquelle 10 vorgesehen, die einen Lichtstrahl nach unten auf einen zu prüfenden Gegenstand, wie z.B. ein Stück Schnittholz, 12 wirft. Obwohl die Darstellung nach Fig. 1 eine senkrechte Beleuchtung zeigt, ist es auch möglich, Schrägbeleuchtung einzusetzen, um Oberflächenunebenheiten, wie Löcher, besser erfassen zu können. Das Stück Schnittholz 12 ist in seiner Längsrichtung beweglich und wird auf nicht dargestellten Führungseinrichtungen in Richtung des Pfeils 14 mit einer Geschwindigkeit von 3 m/s bewegt. Der Lichtstrahl aus der Lichtquelle 10 ist auf der Oberfläche des Holzstücks 12 fokussiert, wobei der Lichtstrahl quer zur Hauptbewegungsrichtung, also zur Längsrichtung des Holzstücks 12, kegelförmig aufgeweitet ist, während er in Längsrichtung des Holzstücks 12 stärker fokussiert ist. Dadurch wird eine relativ hohe Lichtintensität erzielt.

Die Lichtquelle 10 weist einen derartigen Abstand von der Oberfläche 16 des Holzstücks 12 und damit gegebenenfalls auch von der Fehlerstelle 15 auf, daß eine gleichmäßige Ausleuchtung erzielt wird. Unmittelbar benachbart zu der Lichtquelle 10 ist eine optische Erfassungsvorrichtung 18 angeordnet. Die optische Erfassungsvorrichtung 18 weist ein Objektiv 20 auf, das ebenfalls auf die Oberfläche 16 fokussiert ist. Die Anordnung von Lichtquelle 10 und Objektiv 20 zueinander ist so gewählt, daß keine Meßfehler, insbesondere keine Parallaxenfehler auftreten. Dann kann auch bei einer relativ großen Transportgeschwindigkeit des Holzes eine Auflösung in der Größenordnung von 1 mm erzielt werden.

Während der Bewegung des Holzes wird die Oberfläche 16 von der optischen Erfassungsvorrichtung 18 zeilenweise abgetastet. Pro Zeile stehen dabei 0,3 m/s zur Verfügung. Für die Umwandlung der optischen Signale in die elektrischen Signale kommen daher schnelle Halbleiter-Zeilenkameras infrage. Bei reduzierten Geschwindigkeits- bzw. Auflösungs-Anforderungen können aber auch preiswertere optoelektrische Wandler Verwendung finden.

Es besteht die Möglichkeit, ein optisches und/oder ein elektrisches Filter für die optische Erfassungsvorrichtung 18 vorzusehen. Damit können Störeinflüsse und Verfälschungen des Meßergebnisses ausgeblendet werden.

Wie Fig. 1 weiter zeigt, ist im Vorschubbereich des zu erfassenden Schnittholzes 12 ein Tragrahmen 100 angeordnet, der · Bestandteil der Prüfvorrichtung ist und die Erfassungsvorrichtung 18 und die Lichtquelle 10 aufnimmt. Dieser Tragrahmen 100 kann ein U-förmiges Profil mit seitlichen Längsholmen, die dann gleichzeitig die Standfüße des Tragrahmens bilden, und einen oberen Querholm aufweisen, der dann die Erfassungsvorrichtung 18 mit der Lichtquelle 10 trägt, wenn die Oberfläche 16 des Schnittholzes 12 erfaßt werden soll. Sollten die Seitenflächen 16a,16b erfaßt werden, dann sind an den senkrechten Längsholmen des Tragrahmens Erfassungsvorrichtungen und Lichtquellen vorgesehen. Soll auch die untere Fläche 16c des Schnittholzes 12 erfaßt werden, so wird ein ringförmiger Tragrahmen 100, wie in Fig. 1 dargestellt, zur Anwendung gelangen, der dann auch an seinem unteren Querholm eine Erfassungsvorrichtung und eine Lichtquelle trägt. Die Zuführung des zu prüfenden Gegenstandes in die Prüfvorrichtung kann einerseits durch direktes Einbringen des zu prüfenden Gegenstandes oder vermittels einer Zulaufbahn 150 erfolgen, die als umlaufend angetriebenes Transportband ausgebildet ist.

Wenn mehr als eine Fläche des Schnittholzes 12 erfaßt werden soll, braucht nicht jede Schnittholzfläche mittels einer separaten Lichtquelle beleuchtet zu werden. Vielmehr kann in dem Strahlengang der Lichtquelle an dem Tragrahmen 100 eine entsprechende Anzahl von Umlenkspiegeln 110 derart angeordnet sein, daß eine oder auch mehrere Flächen des Schnittholzes beleuchtet werden können. Bei dem in Fig. 1 dargestellten Ausführungsbeispiel wird mittels der an dem Tragrahmen 100 angebrachten Umlenkspiegel die Oberfläche 16 des Schnittholzes beleuchtet. Werden über eine Lichtquelle 10 mehrere Flächen des Schnittholzes beleuchtet, dann ist jeder beleuchteten Schnittholzfläche eine Erfassungsvorrichtung 18 zugeordnet. Es besteht aber auch die Möglichkeit, über ein Umlenkspiegelsystem mit nur einer einzigen Erfassungsvorrichtung 18 mehrere Schnittholzflächen zu erfassen. Über unterschiedliche Lichtintensitäten, Länge des zurückgelegten Lichtstrahles oder Beleuchtungsstrahles können die einzelnen Schnittholzflächen festgelegt und einzeln erfaßt und die erfaßten Werte so gespeichert werden, daß sie den einzelnen Schnittholzflächen später zugeordnet werden können, wobei auch die Möglichkeit besteht, in - schneller Zeitfolge in wiederkehrenden Reihenfolgen die einzelnen verschiedenen Schnitholzflächen zu erfassen.

Als Lichtquellen 10 können alle geeigneten Lichtquellen verwendet werden, insbesondere gleichgerichtetes Licht, monochromatisches Licht, Laserlicht od.dgl.

Der die Erfassungsvorrichtung 18 und die Lichtquelle 10 aufnehmende Tragrahmen 100 nimmt darüber hinaus auch alle weiteren Teile der Prüfvorrichtung auf. Die Meßschaltung ist dann in einem Gehäuse 115 angeordnet, das mit dem Tragrahmen 100 verbunden sein kann.

In der in Fig. 2 dargestellten Ausführungsform einer Meßschaltung ist ein CCD-Scanner 22 als Wandler vorgesehen. Der CCD-Scanner 22 ist über ein geeignetes Interface 24 mit einem Mikrocomputer 26 verbunden, der eine zentrale Verarbeitungseinheit CPU, einen Schreib/Lese-Speicher RAM und einen Programmspeicher ROM aufweist. Das Interface 24 dient als Anpaßschaltung zwischen dem CCD-Scanner 22 und dem Mikrocomputer 26. Der Mikrocomputer 26 weist ferner eine Bedieneinheit 28 auf, die die Anzeige der gemessenen Fehlstellen erlaubt und ·die Eingabe von Parame tern für die Steuerung der Fehlererfassung ermöglicht. Ferner ist ein Drucker 30 vorgesehen, der dazu geeignet ist, ein Protokoll der geprüften Hölzer zu liefern, das zugleich zur Erfassung des Eingangs in das Lager dienen kann.

Bevorzugt findet ein CCD-Scanner 22 mit 500 bis 2000 Pixel Verwendung. Mit einem derartigen Scanner ist es möglich, auch größere Holzoberflächen abzutasten. Die Abtastung selbst erfolgt in der bekannten Weise, so daß das Eingangssignal für das Interface 24 in serieller Form vorliegt. In dem Interface 24 wird eine A/D-Wandlung vorgenommen. Das jeder Abtastperiode zugehörige Meßsignal wird in digitalisierter Form dem Mikrocomputer 26 zugeführt. Der Mikrocomputer 26 prüft, ob innerhalb der gemessenen CCD-Zeile ein Meßwert die Fehlerwertschwelle überschreitet. Besonders vorteilhaft ist es hierbei, wenn eine bei lediglich einem einzelnen Pixel auftretende Fehlerwertanzeige ausgeblendet wird, da es sich dann höchstwahrscheinlich lediglich um eine Reflexion einer Holzfaser handelt.

Ferner ist eine Auflösung von 8 bit für den A/D-Wandler ausreichend, so daß auf preiswerte Technik zurückgegriffen werden kann. Der Mikrocomputer kann auch als Single-Chip-Baustein mit integrierter Ansteuerung der Peripheriegeräte ausgebildet sein, was eine weitere Kostenreduzierung ermöglicht.Zur Sicherstellung eines störungsfreien Betriebes auch unter rauhen Umweltbedingungen kann die Elektronik in -zudem stromsparender - CMOS-Technik ausgeführt sein.

Der in dem Mikrocomputer 26 verwendete Halbleiterspeicher RAM kann sowohl zur Zwischenspeicherung der erfaßten Fehlerstellen unter Berücksichtigung ihrer Größe und Lage als auch als reiner CPU-bezogener Arbeitsspeicher ausgebildet sein. Die erfindungsgemäße Meßschaltung kann auch als Front-End-Prozessor in einem Netzwerk, besonders vorteilhaft mit Sternstruktur, eingesetzt werden. Im Stand-Alone-Betrieb kommt jedoch auch der Einsatz eines zusätzlichen Hintergrundspeichers in Betracht.

In Fig. 3 ist eine weitere Ausführungsform der erfindungsgemäßen Meßschaltung vorgesehen. In dieser Ausführungsform wird anstelle des CCD-Scanners eine Bildaufnahmeröhre VDK als optische Erfassungsvorrichtung 18 eingesetzt. Das Ausgangssignal der optischen Erfassungsvorrichtung 18 wird einem Spannungsteiler $P_1$ zugeleitet, dessen Ausgang mit dem nichtinvertierenden Eingangsanschluß eines Komparators 30 verbunden ist. Der invertierende Eingangsanschluß des Komparators 30 ist mit dem Ausgang eines Potentiometers $P_2$ verbunden. Das Potentiometer $P_2$ dient zur Grundeinstellung des Helligkeitswertes der verwendeten Holzart bzw zur Anpassung an die verwendete Lichtquelle. Das Potentiometer $P_2$ ist ferner in Reihe mit einem Stellglied 32 geschaltet, das beispielsweise als FET-Transistor ausgebildet sein kann. Vermittels des Stellglieds 32 ist eine automatische Anpassung an die verwendete Holzart möglich. Ferner ist das Potentiometer $P_2$ über einen Widerstand $R_1$ mit Masse verbunden.

Der Eingangsanschluß des Stellgliedes 32 und der Ausgangsanschluß des Komparators 30 sind über einen integrierten D/A-A/D-Wandler 34 mit dem Mikrocomputer 26 verbunden.

Der Spannungsteiler $P_1$ dient zur Einstellung der Empfindlichkeit der erfindungsgemäßen Meßschaltung für Fehler, also zur Einstellung des Fehler-Schwellenwertes. Ein weiterer Spannungsteiler wird durch ein Potentiometer $P_3$ und einen Widerstand $R_2$ zwischen der Betriebsspannung + $U_B$ und Masse gebildet. Der Spannungsteiler $P_1$ ist mit seinem "kalten" Ende mit dem Mittelpunkt des durch P₃ und R₂ gebildeten Spannungsteilers verbunden. Dadurch wird erreicht, daß die Fehlerschwelle in Abhängigkeit von der Einstellung des Spannungsteilers P₁ relativ, also unabhängig von dem Absolutwert der eingestellten Helligkeit, festgelegt werden kann.

Die in Fig. 3 dargestellte Ausführungsform der Meßschaltung erlaubt die vollautomatische Prüfung von Schnittholz, nachdem zunächst eine gerätespezifische Justierung der Trimm-Potentiometer P₃ und P₂ vorgenommen worden ist. Das Stellglied 32 wird auf einen Wert eingestellt, der der Grundhelligkeit des verwendeten Holzes entspricht. Hierzu wird zunächst in der Einstell-Betriebsart des Mikrocomputers 26 ein fehlerfreies oder nahezu fehlerfreies Stück Holz der zu überprüfenden Art gemessen. Dessen Helligkeits-Durchschnittswert wird von dem Mikrocomputer 26 berechnet und für die Grundeinstellung übernommen. Dann wird in die Meß-Betriebsart umgeschaltet und mittels P₁ eine Einstellung der gewünschten Fehlerschwelle vorgenommen. Die Fehlerschwelle ist in der Regel abhängig von der gewünschten Qualitätstoleranz und damit unter anderem von der verwendeten Holzart. Dann kann die ganze Charge mit hoher Geschwindigkeit geprüft und die Fehlerstellen abgespeichert werden.

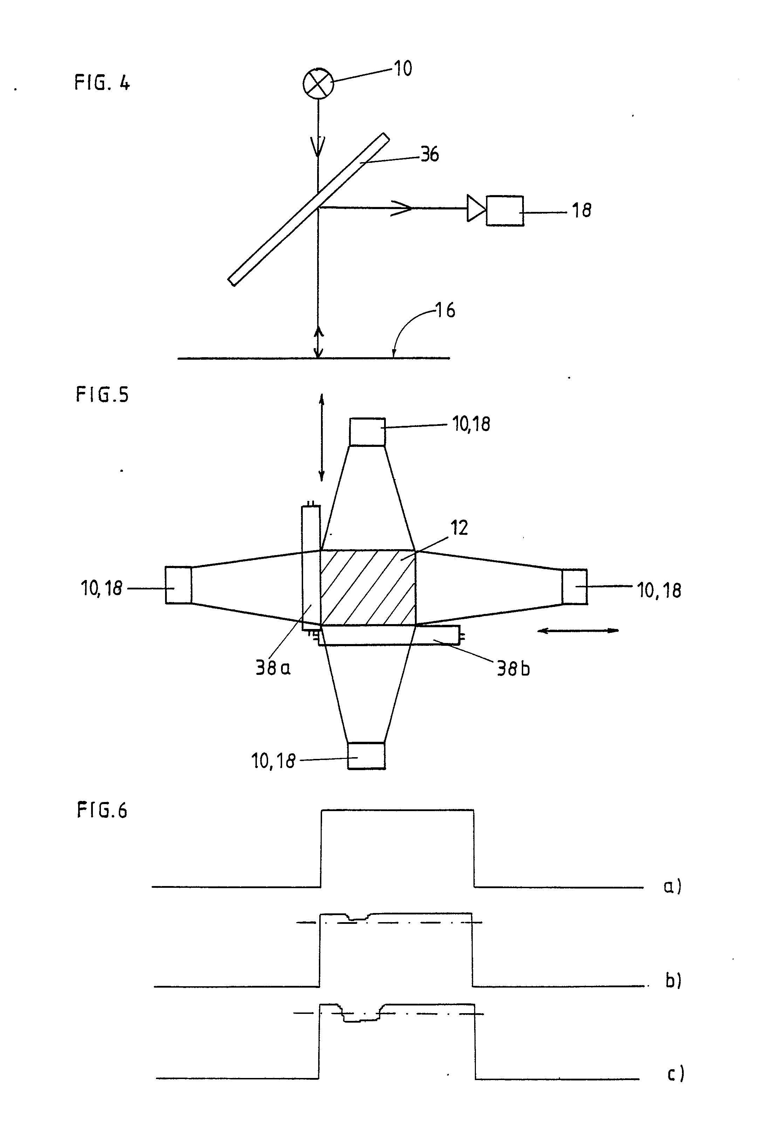

In Fig. 4 ist eine besonders vorteilhafte Ausbildung der optischen Erfassungsvorrichtung 18 in Verbindung mit der Lichtquelle 10 dargestellt. Es wird ein halbdurchlässiger Spiegel 36 verwendet, der im Lichtstrahl von der Lichtquelle 10 zur Oberfläche 16 schräg angeordnet ist. Nach Durchtreten des halbdurchlässigen Spiegels gelangt der von der Lichtquelle 10 kommende Lichtstrahl auf die Oberfläche 16 und wird dort reflektiert. Er trifft auf die Unterseite des halbdurchlässigen Spiegels 36 und wird dort reflektiert und der optischen Erfassungsvorrichtung 18 zugeleitet. Diese Ausführung erlaubt eine besonders genaue Messung der Lage der Fehler.

In Fig. 5 ist eine weitere Ausführungsform der Prüfeinrichtung dargestellt. Das zu prüfende Stück Schnittholz 12 ist ein Vierkantbalken, dessen Kanten alle untersucht werden müssen. Hierzu sind vier Lichtquellen mit vier optischen Erfassungsvorrichtungen je über einer der Schnittkanten angeordnet. Lediglich zwei der Erfassungsvorrichtungen sind mit einer Autofokussiermechanik versehen, da der Abstand zur Holzoberfläche bei den beiden anderen Erfassungsvorrichtungen aufgrund der verwendeten Führungsrollen 38a und 38b stets gleich ist.

In Fig. 6 ist bei a) das Ausgangssignal einer optischen Erfassungsvorrichtung beim einzeiligen Abtasten eines fehlerfreien Holzstücks dargestellt. Bei b) ist eine Fehlerstelle erreicht, die sich durch eine geringere Amplitude in einem Teilbereich des Meßsignals bemerkbar macht. Hier unterschreitet die Amplitude jedoch noch nicht den Fehler-Schwellenwert. Bei c) ist das Meßsignal während des Abtastens einer Meßzeile dargestellt, welche die Fehlerstelle voll erfaßt. Es wird ein Informationssignal abgegeben und die Position der Fehlerstelle abgespeichert. Die längenbezogene Abspeicherung erfolgt dadurch, daß von dem Mikrocomputer 26 die Zeit zwischen der Vorderkante des gerade durchlaufenden Stücks Schnittholz bis zum Auftreten des Informationssignales gemessen wird. Diese Zeit ist wegen der konstanten Vorschubgeschwindigkeit längenproportional und entspricht damit der Länge des fehlerfreien Teils des Holzstücks.

In einer weiteren, nicht dargestellten, Ausführungsform kann es vorgesehen sein, mit einer mit Hammerschlaglack lackierten, glänzenden Oberfläche versehene Gegenstände zu prüfen. Hierzu wird eine Farbkamera eingesetzt, die in Zeilenform erfaßte Informationssignale abgibt. Ein videoschneller Spezialprozessor nimmt eine Vorverarbeitung und Merkmalsextraktion vor. Hammerschlaglack ist insbesondere deswegen schwierig zu prüfen, da es sich um eine einerseits glänzende Oberfläche handelt, andererseits eine zufallsbedingt mehr oder weniger regelmäßige Struktur vorliegt. Bei derartigen Gegenständen besteht die Prüfaufgabe häufig darin, einerseits Farbfehler festzustellen, andererseits korrosionsbegünstigende Risse in der Lackstruktur festzustellen. Durch Schrägbeleuchtung werden Schatten geworfen, die eine Unterscheidung zwischen einem Riß und den von der Kamera als dunkel interpretierten Stellen der Hammerschlaglackierung ermöglichen. Zur Feststellung von Rissen in der Lackoberfläche und zur Feststellung von Farbfehlern ist also eine - schwellwertorientierte Verarbeitung möglich.

Mit einer anderen Ausführungsform der erfindungsgemäßen Prüfvorrichtung lassen sich Dragéstreifen oder Korkplatten auf unterschiedliche Fehler prüfen. Bei Dragéstreifen, die aus Aluminiumstreifen mit einer in regelmäßiger Verteilung darauf befindlichen und je einzeln von Klarsichtfolie umschweißten Tabletten bestehen, können beispielsweise Verpackungsfehler auftreten, oder es können einzelne Tabletten fehlen. Aufgrund der Reflexion der Klarsichtfolie müßten geringe Grauwertunterschiede erfaßt werden können. Dementsprechend werden für die Bereitstellung des Informationssignales Kameras oder andere optische Erfassungsvorrichtungen mit hoher Auflösung verwendet.

Bei Korktapete wird eine gleichmäßige Verteilung der Hell-und Dunkelstellen gefordert. Die Einordnung in verschiedene Qualitätsklassen erfordert aufgrund der schnellen Verarbeitung, wie auch in dem letztgenannten Anwendungsbeispiel, die Bereitstellung von schnellen Spezialprozessoren. Besonders vorteilhaft lassen sich hierbei mehrere Prozessoren in Parallelverarbeitung installieren, da alle Verarbeitungs-und Übertragungsvorgänge synchronisiert ablaufen können.

Die Videokamera wird bei den letztgenannten Anwendungsbeispielen zweckmäßigerweise über ein speziell ausgebildetes Interface 24 angeschlossen. Dieses weist den A/D-Wandler auf, wobei mit einer -an sich bekannten -Lösung unter Verwendung eines EPROMS eine Korrektur der Kennlinie erfolgen kann, um diese hinsichtlich Nullpunkt und Steilheit zu linealisieren. Anstelle des EPROMS kann bei unterschiedlichen verwendeten Konfigurationen ein von der Rechenvorrichtung ladbarer Schreib/Lesespeicher RAM zur Shading-Korrektur verwendet werden. Wahlweise können an dieses Interface dann anstelle der Video-Kamera auch Laser-Scanner angeschlossen werden.

**Ansprüche**

1. Prüfvorrichtung für die Erfassung unterschiedlich ausgestalteter Oberflächen von Gegenständen, wie keramische Erzeugnisse, Gegenstände aus Glas, Holz, Kunststoffen, in Klarsichtfolie verpackte Gegenstände u.dldg., dadurch gekennzeichnet, daß der zur prüfende Gegenstand (12) relativ zu der Prüfvorrichtung in einer Hauptbewegungsrichtung bewegbar ist, daß der Gegenstand (12) in einer Ebene quer zur Hauptbewegungsrichtung (Pfeil 14) mit im wesentlichen gleicher Intensität beleuchtbar ist und daß eine in dieser Ebene angeordnete, die reflektierten Helligkeits-und/oder Farbwerte des Gegenstandes in Zeilenform erfassende optische Erfassungsvorrichtung (18), ansprechend auf Helligkeits-und/oder Farbänderungen in der von der Erfassungsvorrichtung (18) erfaßten beleuchteten Ebene des Gegenstandes, während der Relativbewegung von Gegenstand und optischer Erfassungsvorrichtung ein Informationssignal abgibt.

2. Prüfvorrichtung nach Anspruch 1, dadurch gekennzeichnet, daß die optische Erfassungsvorrichtung (18) mit einer das Informationssignal interpretierenden Rechenvorrichtung (26) verbunden ist, die auftretende Besonderheiten in der Oberfläche der geprüften Gegenstände erfaßt und anzeigt, wobei die Anzeige durch Abspeicherung und/oder Signalgebung erfolgt.

3. Prüfvorrichtung nach Anspruch 2, dadurch gekennzeichnet, daß die Rechenvorrichtung (26) eine Speichervorrichtung aufweist, in welcher auftretende Besonderheiten als Informationssignale sequentiell abspeicherbar sind.

4. Prüfvorrichtung nach einem der Ansprüche 2 oder 3, dadurch gekennzeichnet, daß die Rechenvorrichtung eine Zeitmeßeinrichtung aufweist, die die Zeitdauer vom Erfassen der Vorderkante, in der Hauptbewegungsrichtung gesehen, eines Gegenstandes bis zum Auftreten der Besonderheit mißt, und daß die Zeitmeßeinrichtung mit einer Speichervorrichtung verbunden ist.

5. Prüfvorrichtung nach einem der vorhergehenden Ansprüche, dadurch gekennzeichnet, daß eine Anpaßschaltung (Interface 24) vorgesehen ist, mit welcher zumindest eine Erkennungsschwelle für das Interformationssignal einstellbar ist.

6. Prüfvorrichtung nach Anspruch 5, dadurch gekennzeichnet, daß die Anpaßschaltung (Interface 24), insbesondere elektronische, Steuerelement ($P_2$ ,$R_1$ ,32) für die Anpassung der optischen Erfassungsvorrichtung (18) an unterschiedliche Grundhelligkeiten der Oberfläche des Gegenstandes oder der Lichtquelle zur Ausnutzung der maximalen Dynamik der optischen Erfassungsvorrichtung aufweist.

7. Prüfvorrichtung nach einem der Ansprüche 2 bis 6, dadurch gekennzeichnet, daß die optische Erfassungsvorrichtung (18) einen Filter für die Erhöhung der Empfindlichkeit auf bestimmte Besonderheiten aufweist.

8. Prüfvorrichtung nach einem der vorhergehenden Ansprüche 1 bis 7, dadurch gekennzeichnet, daß eine Lichtquelle (10) vorgesehen ist, mit welcher der Gegenstand in der Ebene quer zur Hauptbewegungsrichtung (Pfeil 14) kegelförmig oder in abgestimmter Geometrie beleuchtbar ist, wobei die Lichtquelle einen schmalen Bereich beleuchtet, der sich über die ganze Länge des Gegenstandes in der Ebene erstreckt, wobei die Lichtquelle insbesondere monochromatisches oder gleichgerichtetes Licht oder Laserlicht od.dgl. aussendet.

9. Prüfvorrichtung nach einem der vorhergehenden Ansprüche, dadurch gekennzeichnet, daß mit der optischen Erfassungsvorrichtung (18) eine punktförmige oder Array-Abtastung, insbesondere in einem Zeilenraster, vornehmbar ist.

10. Prüfvorrichtung nach einem der vorhergehenden Ansprüche 1 bis 9, dadurch gekennzeichnet, daß ein Tragrahmen (100) vorgesehen ist, an dem eine obere und/oder zwei seitliche und/oder eine untere optische Erfassungsvorrichtung (18), insbesondere mit je einer Lichtquelle (10), angeordnet sind.

11. Prüfvorrichtung nach einem der Ansprüche 1 bis 9, dadurch gekennzeichnet, daß die Vorrichtung einen Tragrahmen (100) aufweist, der eine Lichtquelle (10) und eine optische Erfassungsvorrichtung (8) trägt und an welchem eine Anzahl von Umlenkspiegeln (110) derart angeordnet sind, daß die Lichtquelle (10) den Gegenstand (12) mit im wesentlichen gleicher Intensität beleuchtet, wobei die optische Erfassungsvorrichtung (18) über das Umlenkspiegelsystem die beleuchtete Ebene des Gegenstandes erfassend ist.

12. Prüfvorrichtung nach einem der vorhergehenden Ansprüche 1 bis 11, dadurch gekennzeichnet, daß als Gegenstand z.B. Schnittholz in Brett- oder Balkenform mit im wesentlichen zueinander parallelen Seitenflächen verwendet wird.

13. Verfahren zum Prüfen von Gegenständen mit unterschiedlich ausgestalteter Oberfläche, wie keramische Erzeugnisse, Gegenstände aus Glas, Holz, Kunststoffen, in Klarsichtfolie verpackte Gegenstände u.dgl., dadurch gekennzeichnet, daß die Gegenstände kontinuierlich in einer Hauptbewegungsrichtung relativ zu einer optischen Erfassungsvorrichtung durch Absolutbewegung der optischen Erfassungsvorrichtung oder der Gegenstände bewegt werden, daß ein schmaler und sich über die Breite des Gegenstandes erstreckender Bereich in einer sich quer zur Hauptbewegungsrichtung erstrekkenden Ebene beleuchtet wird und daß die optische Erfassungsvorrichtung in der Ebene quer zur Bewegungsrichtung des Gegenstandes arbeitet und ansprechend auf Helligkeits-und/oder Farbänderungen des Gegenstandes während der Relativbewegung von Gegenstand und optischer Erfassungsvorrichtung ein Informationssignal abgibt.

14. Verfahren nach Anspruch 13, dadurch gekennzeichnet, daß die ganze Breite des Gegenstandes in einem Punktraster zeilenförmig von der Erfassungsvorrichtung erfaßt wird und daß das in Zeilenform vorliegende Informationssignal einem Rechner zugeleitet wird, der jeden Punkt oder Punktgruppen auswertet und in Echtzeit Eigenschaften der Gegenstände feststellt, wobei insbesondere auch eine Array-Abtastung anwendbar ist.

15. Verfahren nach Anspruch 12 oder 13, dadurch gekennzeichnet, daß das Informationssignal positionsbezogen zu jedem geprüften Gegenstand, insbesondere zu jedem Stück Schnittholz, abgespeichert wird.

16. Verfahren nach einem der Ansprüche 13 bis 15, dadurch gekennzeichnet, daß die zu erfassende Oberfläche des zu prüfenden Gegenstandes so beleuchtet wird, daß der von einer Lichtquelle abgegebene Lichtstrahl einen Winkel gegenüber dem von der zu prüfenden Oberfläche reflektierten und von der optischen Erfassungs vorrichtung erfaßten Licht bildet, der von 0 verschieden ist, und daß der von den beiden Lichtstrahlen aufgespannte Winkel insbesondere zwischen 30 und 90° beträgt.

0 234 492

FIG.1

FIG.2

FIG.3

FIG. 4

FIG.5

FIG.6

a)

b)

c)